**Europäisches Patentamt**

**European Patent Office**

**Office européen des brevets**

(11) Veröffentlichungsnummer: **0 259 769**

A1

(12) **EUROPÄISCHE PATENTANMELDUNG**

(21) Anmeldenummer: 87112791.6

(22) Anmeldetag: 02.09.87

(51) Int. Cl.⁴: **H02N 11/00** , A61N 5/00 , A61N 1/16 , H05F 7/00 , A23L 3/26

(30) Priorität: 08.09.86 DE 3630523

(43) Veröffentlichungstag der Anmeldung: 16.03.88 Patentblatt 88/11

(84) Benannte Vertragsstaaten: AT BE CH DE ES FR GB GR IT LI LU NL SE

(71) Anmelder: **Oehme, Rainer**
**Moorstrasse 10**
**D-3033 Schwarmstedt(DE)**

(72) Erfinder: **Oehme, Rainer**
**Moorstrasse 10**
**D-3033 Schwarmstedt(DE)**

(74) Vertreter: **Schlagwein, Udo, Dipl.-Ing.**
**Anwaltsbüro Ruppert & Schlagwein**
**Bahnhofsallee 11**
**D-6350 Bad Nauheim(DE)**

(54) **Pyramidenenergieanlage.**

(57) Ein Leiter (2) verläuft in Form einer kegligen Spirale außenseitig über eine Pyramide (1). Ihr abführender Leiterteil (4) ist über die Spitze der Pyramide (1) geleitet. Bei dem Leiter (2) handelt es sich entweder um einen elektrischen Leiter, oder aber um einen Rohrkörper, durch den ein zu behandelndes Medium geführt werden kann.

Mit einem solchen Leiter (2) als Energieaufnahmeeinrichtung läßt sich das gesamte Schwingungsspektrum der Pyramide (1) erfassen.

Fig. 1

EP 0 259 769 A1

## Pyramidenenergieanlage

Die Erfindung bezieht sich auf eine Pyramidenenergieanlage mit einer außenseitig auf der Pyramide vorgesehenen Energieaufnahmeeinrichtung. Eine solche Pyramidenenergieanlage ist in der DE-OS 35 39 610.5-33 beschrieben.

Bei der bekannten Pyramidenenergieanlage erfolgt die Energieaufnahme mittels eines von oben her auf die Pyramide aufgesetzten Schwingtopfes. Elektrische Leitungen führen die Energie dann zu Chakra-Elektroden weiter. Ein solcher Schwingtopf, der üblicherweise aus Messing besteht, ist teuer in seiner Herstellung und hat funktionell den Nachteil, daß von ihm nicht das gesamte Schwingunsspektrum von der Basis bis zur Spitze der Pyramide erfaßt wird.

Der Erfindung liegt die Aufgabe zugrunde, eine Pyramidenenergieanlage der eingangs genannten Art derart auszubilden, daß mit ihrer Energieaufnahmeeinrichtung das gesamte Schwingungsspektrum der Pyramide erfaßt werden kann.

Diese Aufgabe wird erfindungsgemäß dadurch gelöst, daß die Energieaufnahmeeinrichtung ein wendelförmig um die gesamte Pyramide herum geführter Leiter ist.

Durch diese Gestaltung wird mit sehr einfachen Mitteln in einem sehr hohen Maße Pyramidenenergie aufgenommen. Das erfolgt ohne zwischengeschaltete Resonanz. Die Energie wird unmittelbar in den Leiter eingespeist. Weiterhin wird das gesamte Schwingungsspektrum von der Basis bis zur Spitze der Pyramide erfaßt. Da die Energieaufnahmeeinrichtung ein heranführendes und ein wegführendes Leiterende hat, stehen zwei Anschlüsse für die Weiterleitung der Pyramidenenergie zur Verfügung. Dadurch kann die Pyramidenenergieanlage besonders vorteilhaft in Medizin und Technik eingesetzt werden. Es ist beispielsweise möglich, mit einer solchen Pyramidenenergieanlage im Durchlaufverfahren für den Gartenbau und in der Land-, Forst- und Wasserwirtschaft Wasser durch Pyramidenenergie zu vitalisieren. Durch Besprühen mit mit Pyramidenenergie aufgeladenem Wasser können Landflächen in ihrer unnatürlichen Radioaktivität herabgesetzt werden. Messungen mit dem Geigerzähler haben gezeigt, daß radioaktiv belastetes Wasser, das der Einwirkung von Pyramidenenergie ausgesetzt wurde, danach erheblich geringere, radioaktive Werte aufwies.

Auch Treibstoffe oder Lebensmittel, zum Beispiel Getränke, können im Durchlaufverfahren in ihrer radioaktiven Belastung herabgesetzt werden.

Durch die erfindungsgemäße Pyramidenenergieanlage steht Pyramidenenergie für medizinische, biologische und technische Zwecke kostenlos und in unbegrenzter Menge zur Verfügung.

Besonders einfach ist die Energieaufnahmeeinrichtung gestaltet, wenn der Leiter die Form einer kegelförmigen Spirale hat.

Zur weiteren Vereinfachung trägt es bei, wenn der Leiter auf den Kanten der Pyramide aufliegt.

Das Aufbauen einer Pyramidenenergieanlage am Einsatzort ist besonders rasch durchzuführen, wenn gemäß einer anderen Ausgestaltung der Erfindung der wendelförmig gebogene Leiter durch Bänder, Seile oder ähnliches so miteinander verknüpft bzw. verbunden ist, daß seine Form beim Abheben von der Pyramide bestehen bleibt.

Eine ebenfalls sehr einfache, abgeänderte Ausführungsform der Erfindung besteht darin, daß der Leiter innenseitig entlang eines über die Pyramide gesetzten, zylindrischen Stützringes - schraubenlinienförmig gewickelt verläuft.

Der Leiter ist einfacher zu wickeln, wenn er außenseitig entlang eines über die Pyramide gesetzten, zylindrischen, für die Pyramidenenergie durchlässigen Stützringes schraubenförmig gewickelt verläuft.

Die von der Pyramidenspitze nach oben abgestrahlte Energie wird im besonders hohen Maße aufgenommen, wenn gemäß einer ganz besonders vorteilhaften Ausgestaltung der Erfindung der Leiter mit seinem die Energie ableitenden Leitungsteil über die Spitze der Pyramide geführt ist.

Grundsätzlich kann der um die Pyramide herum geführte Leiter entweder ein elektrisches Kabel oder ein Rohrkörper sein. Im ersten Fall kann man dann die aufgenommene Energie über elektrische Leitungen beispielsweise zu Chakraelektroden führen. Im zweiten Fall wird es möglich, durch den als Rohrkörper ausgebildeten Leiter beispielsweise Flüssigkeiten fließen zu lassen, so daß diese unmittelbar Pyramidenenergie aufnehmen.

Die Erfindung läßt zahlreiche Ausführungsformen zu. Drei davon sind in der Zeichnung dargestellt und werden nachfolgend beschrieben. Diese zeigt in

Fig. 1 eine Seitenansicht einer erfindungsgemäßen Pyramidenenergieanlage,

Fig. 2 eine Draufsicht auf die Anlage gemäß Fig. 1,

Fig. 3 eine Seitenansicht einer zweiten Ausführungsform einer Pyramidenenergieanlage,

Fig. 4 eine Draufsicht auf die Anlage gemäß Fig. 3,

Fig. 5 einen Querschnitt durch eine weitere Ausführungsform einer Pyramidenenergieanlage,

Fig. 6 eine Draufsicht auf die Anlage gemäß Fig. 5,

Fig. 7 einen Querschnitt durch eine weitere Ausführungsform einer Pyramidenenergieanlage,

Fig. 8 eine Draufsicht auf die Anlage gemäß Fig. 7.

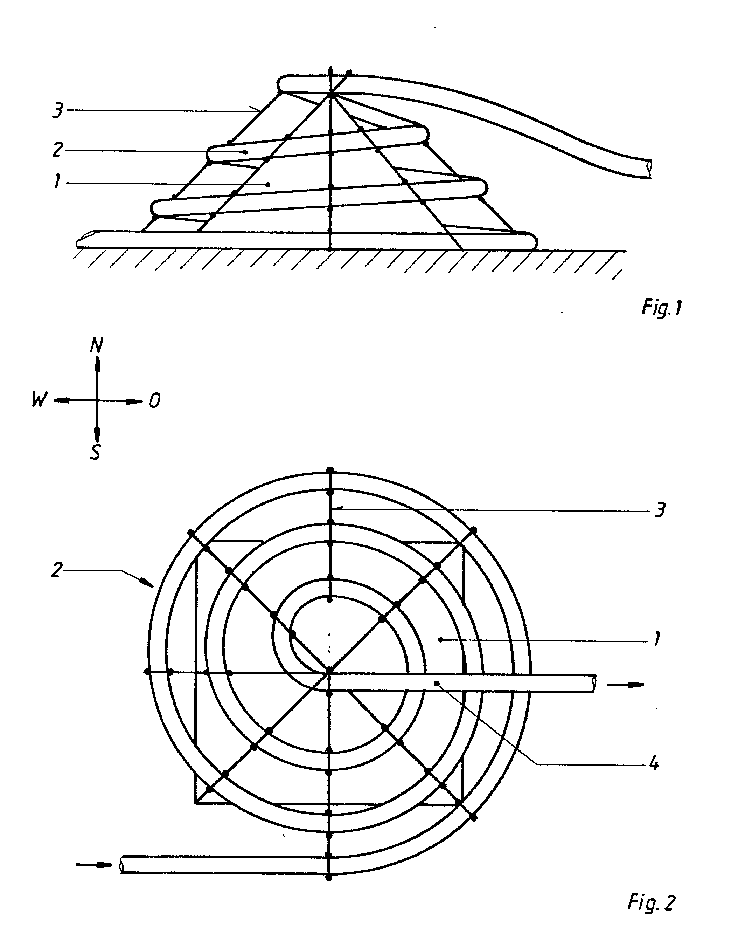

Die Figuren 1 und 2 zeigen eine Pyramide 1, welche mit einer Grundkante in Nord-Süd-Richtung ausgerichtet ist. Um diese Pyramide ist ein Leiter 2 zu einer kegelförmigen Spirale von unten nach oben entgegengesetzt dem Uhrzeigersinn derart gewickelt, daß dieser auf ihren Kanten aufsitzt. Bei diesem Leiter 2 kann es sich um ein ein-oder mehradriges, nicht abgeschirmtes Elektrokabel oder aber um einen für Pyramidenenergie durchlässigen Rohrkörper handeln, durch den eine Flüssigkeit (zum Beispiel Wasser, Milch, Säfte, Treibstoff) oder ein Gas durchgeleitet wird. Die durch den Leiter 2 gebildete Spirale ist durch Bänder 3, Seile oder ähnliches in ihren Windungen so untereinander verknüpft, daß die Leiterspirale beim Abheben von der Pyramide 1 bestehen bleibt. Der von der Pyramide 1 wegführende Leiterteil 4 ist über die Pyramidenspitze geführt, damit er die dort abgestrahlte Energie aufzunehmen vermag.

Die von der Pyramide abgestrahlten Energien treffen auf die Windungen des Leiters 2, der sie aufnimmt und entweder weiter an das ihn durchströmende Medium gibt oder falls der Leiter ein elektrisches Kabel ist, in elektrische Leitungen einspeist, die an ihm angeschlossen sind.

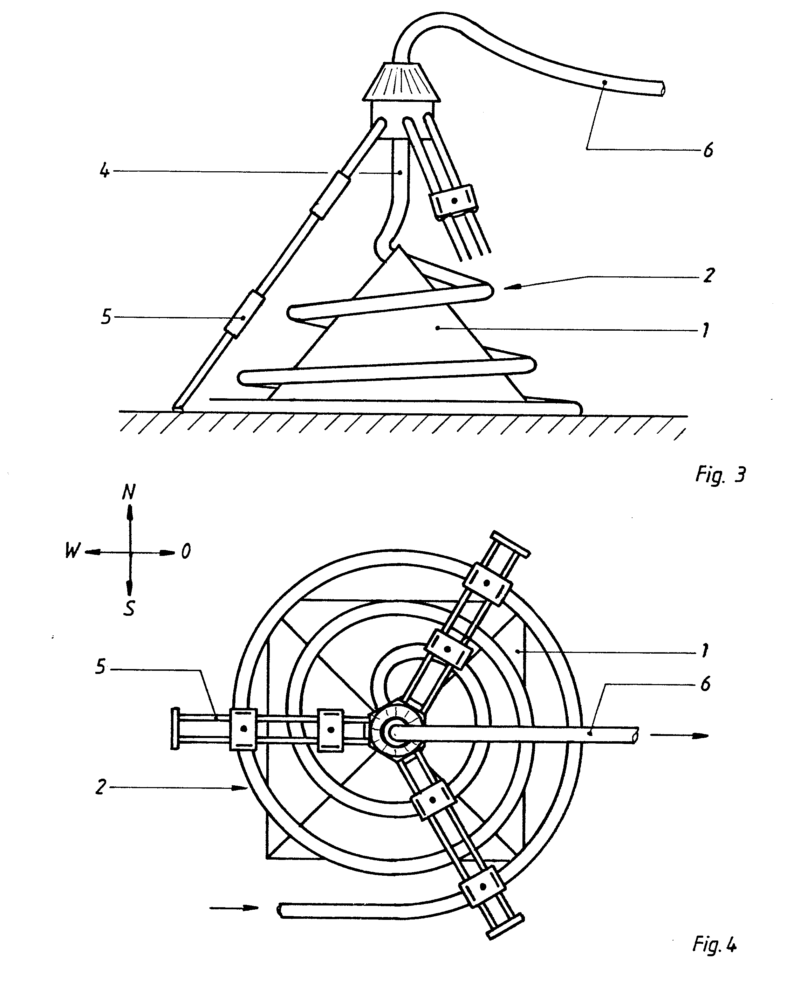

Die Ausführungsform gemäß den Figuren 3 und 4 unterscheidet sich von der zuvor beschriebenen dadurch, daß der Leiter 2 nicht auf den Kanten der Pyramide aufliegt sondern geringen Abstand von diesen hat. Ein handelsüblicher Halter 5 hält den Leiter 2 in der dargestellten Position. Er führt zugleich das Leiterteil 4 oberhalb der Pyramidenspitze senkrecht nach oben, so daß die über die Pyramidenspitze abströmende Pyramidenenergie diesen Leiterteil 4 trifft. Der Halter 5 erlaubt die Pyramidenenergieaufnahme in einem weiten Schwingungsspektrum der Pyramide auch dann, wenn das Leitungsendstück 6 in Bewegung versetzt wird.

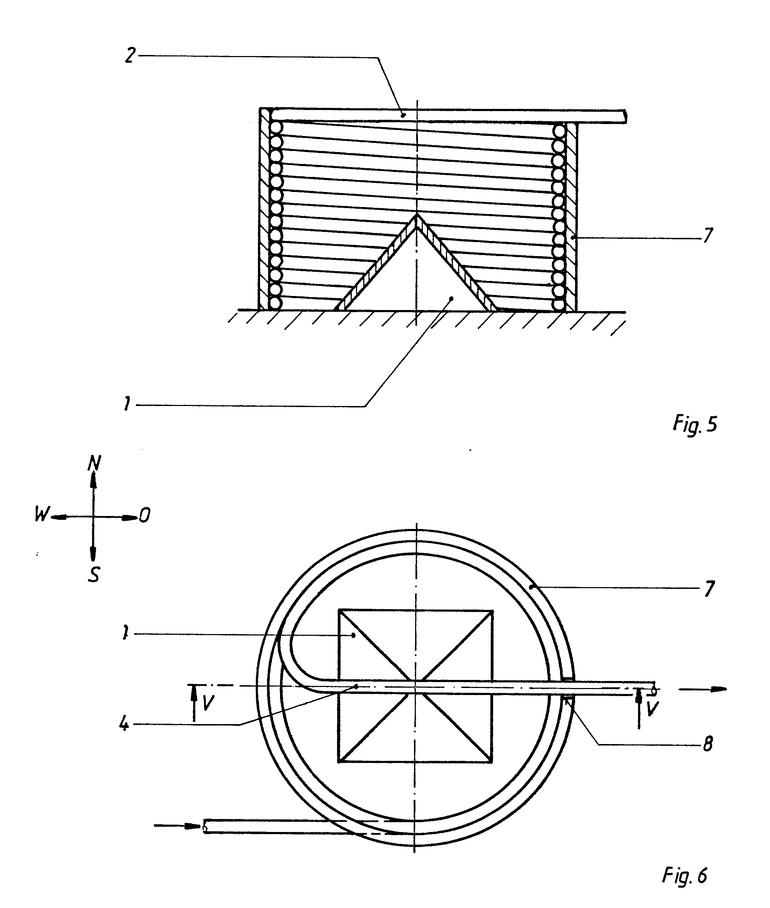

Gemäß den Figuren 5 und 6 ist ein zylindrischer Stützring 7 von oben her über die Pyramide 1 gestülpt. Entlang der Innenwand dieses Stützringes 7 verläuft der Leiter 2 in einer Schraubenlinie. Sein die Energie abführender Leiterteil 4 verläuft wiederum über die Pyramidenspitze und ist mittels einer stirnseitigen Ausnehmung 8 in der Oberseite des Stützringes 7 gehalten.

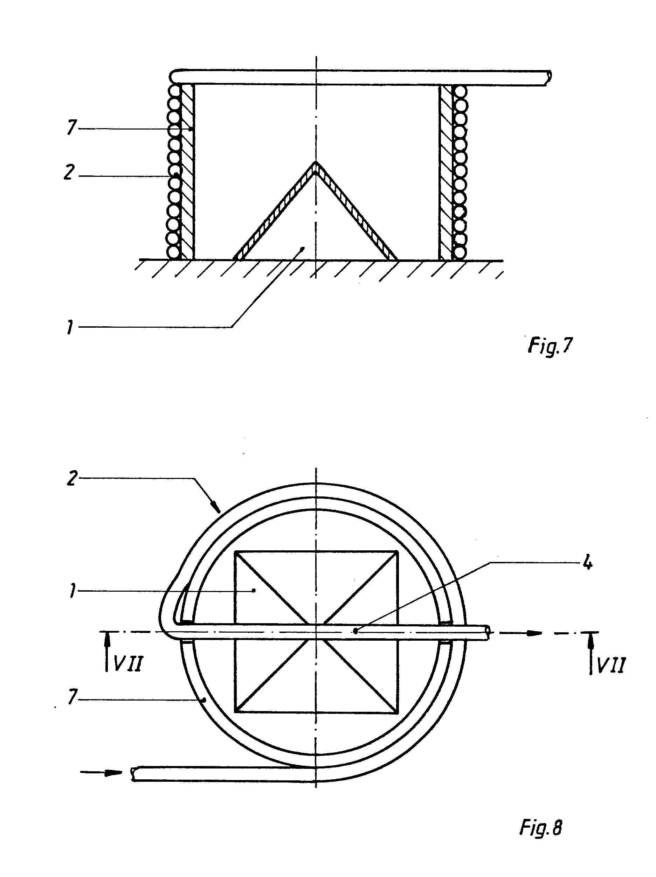

Bei der Ausführungsform gemäß den Figuren 7 und 8 ist der Leiter 2 außenseitig auf den Stützring 7 gewickelt. Der Stützring 7 besteht hierbei aus einem für die Pyramidenenergie durchlässigen Werkstoff. Genau wie bei der vorangegangenen Ausführungsform ist der abführende Leiterteil 4 über die Pyramidenspitze gelegt.

## Ansprüche

1. Pyramidenenergieanlage mit einer außenseitig auf der Pyramide (1) vorgesehenen Energieaufnahmeeinrichtung, dadurch gekennzeichnet, daß die Energieaufnahmeeinrichtung ein wendelförmig um die gesamte Pyramide (1) herum geführter Leiter (2) ist.

2. Pyramidenenergieanlage nach Anspruch 1, dadurch gekennzeichnet, daß der Leiter (2) die Form einer kegelförmigen Spirale hat.

3. Pyramidenenergieanlage nach Anspruch 2, dadurch gekennzeichnet, daß der Leiter (2) auf den Kanten der Pyramide (1) aufliegt.

4. Pyramidenenergieanlage nach einem oder mehreren der vorangehenden Ansprüche, dadurch gekennzeichnet , daß der wendelförmig gebogene Leiter (2) durch Bänder (3), Seile oder ähnliches so miteinander verknüpft bzw. verbunden ist, daß seine Form beim Abheben von der Pyramide (1) bestehen bleibt.

5. Pyramidenenergieanlage nach einem oder mehreren der vorangehenden Ansprüche, dadurch gekennzeichnet, daß der Leiter (2) innenseitig entlang eines über die Pyramide (1) gesetzten, zylindrischen Stützringes (7) schraubenlinienförmig gewickelt verläuft.

6. Pyramidenenergieanlage nach einem oder mehreren der vorangehenden Ansprüche, dadurch gekennzeichnet, daß der Leiter (2) außenseitig entlang eines über die Pyramide (1) gesetzten, zylindrischen, für die Pyramidenenergie durchlässigen Stützringes (7) schraubenförmig gewickelt verläuft.

7. Pyramidenenergieanlage nach einem oder mehreren der vorangehenden Ansprüche, dadurch gekennzeichnet, daß der Leiter (2) mit seinem die Energie ableitenden Leiterteil (4) über die Spitze der Pyramide (1) geführt ist.

8. Pyramidenenergieanlage nach einem oder mehreren der vorangehenden Ansprüche, dadurch gekennzeichnet, daß der Leiter (2) ein elektrisches Kabel ist.

9. Pyramidenenergieanlage nach einem oder mehreren der vorangehenden Ansprüche, dadurch gekennzeichnet, daß der Leiter (2) ein Rohrkörper ist.

3

2

1

Fig. 1

N

W ← → 0

S

3

2

1

4

Fig. 2

*Fig. 3*

*Fig. 4*

Fig. 5

Fig. 6

Fig. 7

Fig. 8

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.3) |
|---|---|---|---|
| A | DE-A-3 320 518 (SCHWEITZER) <br> * Seite 8, Zeilen 8-22; Figur 5 * <br> --- | 1,2,5,6 | H 02 N   11/00 <br> A 61 N    5/00 <br> A 61 N    1/16 <br> H 05 F    7/00 <br> A 23 L    3/26 |
| A | FR-A- 930 466 (PEQUIGNOT) <br> * Seite 2, Zeilen 14-73; Seite 3, Zeilen 11-72; Figuren 1,2 * <br> --- | 1,5,6,9 | |
| A | DE-U-8 535 780 (BRÜGEMANN) <br> * Seite 5, Zeilen 1-33; Figuren 1-4 * <br> --- | 1,2,8 | |
| A,P | DE-A-3 525 521 (KAUSCH) <br> * Spalte 1, Zeile 60 - Spalte 2, Zeile 59; Abbildungen 1-3 * <br> --- | 1 | |
| A | FR-A-1 153 623 (LARONZE) <br> * Insgesamt * <br> --- | 1,8 | |
| D,A P | DE-A-3 539 610 (OEHME) <br> * Anspruch 13; Figur "Blatt 2" * <br> --- | 1,7 | |
| A | CH-B-  97 862 (MASCHINENFABRIK OERLIKON) <br> --- | | RECHERCHIERTE SACHGEBIETE (Int. Cl.3) |
| A | FR-A-2 264 406 (FANTUZZI) <br> --- | | A 61 N <br> A 23 L <br> H 05 F <br> H 02 N |
| A | EP-A-0 168 513 (RUDHARDT) <br> --- | | |
| A | DE-A-2 457 792 (HANSCHMANN) <br> ----- | | |

Der vorliegende Recherchenbericht wurde für alle Patentansprüche erstellt

| Recherchenort | Abschlußdatum der Recherche | Prüfer |
|---|---|---|
| DEN HAAG | 09-12-1987 | SCHMIERER U.J. |